# EUROPÄISCHE PATENTSCHRIFT

(11) **EP 1 150 633 B1**
(45) Veröffentlichungstag und Bekanntmachung des Hinweises auf die Patenterteilung: **31.03.2004**
(21) Anmeldenummer: 00908956.6
(22) Anmeldetag: 29.01.2000
(51) Int. Cl.: A61F 11/08, H04R 1/10

(54) **VORRICHTUNG ZUR SCHALLDÄMMUNG AM MENSCHLICHEN OHR**
DEVICE FOR ATTENUATING SOUND ON THE HUMAN EAR
DISPOSITIF D'ISOLATION ACOUSTIQUE A PLACER SUR L'OREILLE D'UN ETRE HUMAIN

(30) Priorität: 05.02.1999 DE 29902617 U
(43) Veröffentlichungstag der Anmeldung: 07.11.2001
(73) Patentinhaber: Wild, Lars, 31162 Bad Salzdetfurth (DE)
(72) Erfinder: Wild, Lars, 31162 Bad Salzdetfurth (DE)
(74) Vertreter: Lins, Edgar, Dipl.-Phys. Dr.jur.
(86) Internationale Anmeldenummer: PCT/DE2000/000266
(87) Internationale Veröffentlichungsnummer: WO 2000/045760

(56) Entgegenhaltungen:
- EP-A- 0 440 572
- EP-A- 0 590 698
- DE-U- 29 902 617
- US-A- 4 587 965
- US-A- 5 333 622

## Beschreibung

Die Erfindung betrifft eine Vorrichtung zur Vermeidung von Schlafstörungen gemäß dem Oberbegriff des Patentanspruchs 1.

Schlafstörungen oder Schlaflosigkeit stellen in der heutigen Zeit ein zunehmendes und ernsthaftes Problem dar. Die durchschnittliche Schlafdauer hat in den letzten 20 Jahren um 30 Minuten abgenommen. Etwa jeder zehnte Bürger leidet unter extremen Schlafstörungen, die eine medizinische Hilfe erforderlich machen. Die Behandlung von Schlafstörungen erfolgt zumeist in Form der Einnahme von Schlaftabletten, die zum ersten teuer und zum zweiten nicht erfolgverbürgend ist und schließlich auch noch zu gesundheitlichen Schäden führen kann.

In den meisten Fällen hat die Schlaflosigkeit psychische Ursachen. Insbesondere angstbesetzte Empfindungen führen bei vielen Personen zu mitunter chronischen Schlafstörungen. Neben seelischen und organischen Störungen liegt ein weiterer wichtiger Grund für das Auftreten von Schlafstörungen in den durch Lärm oder Geräusche bedingten äußeren Ursachen. Das kann zum einen außerhalb der Wohnung verursachter Lärm, zum Beispiel Straßenlärm, sein. Zum anderen werden lärmbedingte Schlafstörungen durch innerhalb der Wohnung verursachte Geräusche, beispielsweise von Tonwiedergabegeräten, Kindern und anderen Personen, hervorgerufen. Eine über einen längeren Zeitraum anhaltende Geräuschkulisse kann selbst dann noch zu - in diesem Fall psychischen - Schlafstörungen führen, wenn dieser Störfaktor längst ausgeschaltet ist.

Auch Schnarchen und die dadurch beeinträchtigte Atmung können zu Schlafstörungen des Schnarchers führen. Die atemabhängigen Geräusche werden während des Schlafes durch Flatterbewegungen des erschlafften Gaumensegels oder durch Zurücksinken der Zunge hervorgerufen. Häufig ist das Auftreten von Schnarchgeräuschen von der Lagerung des Kopfes im Schlaf abhängig. Rückenlage im Schlaf begünstigt die Geräuschentwicklung.

Auch Dritte werden durch die Schnarchgeräusche in ihrem Schlaf gestört. Beim Schnarchen kann eine Lautstärke von bis zu 88 Dezibel erreicht werden, was etwa der Lautstärke eines direkt neben dem Ohr fahrenden Lastkraftwagens entspricht.

Es sind bereits verschiedene - die Ohren abdeckende oder in diese steckbare - Mittel zu Schalldämmung bekannt, die jedoch insofern nachteilig sind, als die erzielte Geräuschminderung zum einen nicht ausreichend ist und zum anderen verschiedene wichtige Geräusche, beispielsweise der Alarm eines Weckers, einer Alarmanlage oder eines Telefons, oder die bei einem Einbruch auftretenden Geräusche vom Benutzer der Mittel nicht wahrgenommen werden.

Die bereits bekannte, schalldämmenden Mittel beseitigen zwar unter Umständen die geräuschbedingten Schlafstörungen, sind aber dann ursächlich für Schlafstörungen, die durch Ängste hervorgerufen werden. Dazu zählt die Angst, wichtige Geräusche zu überhören. In den meisten Fällen wird daher auf die Benutzung der bereits bekannten, schalldämmenden Mittel gänzlich verzichtet.

Beim Schnarchen sind die bekannten schalldämmenden Mittel ungeeignet, die Schlafstörungen der schnarchenden Person selbst zu beseitigen. Bei einigen scharchenden Personen wird das Schnarchen durch diese Mittel sogar unterstützt, da das eigene Schnarchen nicht mehr wahrgenommen und der Schnarchende nicht mehr angehalten wird, seine Schlafposition zu ändern. Die beeinträchtigte Atmung führt dann weiterhin zu Schlafstörungen.

Auch die Partner von Schnarchenden haben sehr große Schlafstörungen. Die Benutzung der bereits bekannten Mittel zur Schalldämmung wird aus Angst abgelehnt, Phasen der Atemlosigkeit des schnarchenden Partners zu überhören und dann bei einem längeren Atemstillstand nicht reagieren zu können.

Durch EP 0 590 598 A2 sind Hörschutzeinrichtungen bekannt, die mit in den Gehörgang ragenden Ohrstöpseln gebildet sind. In eine Kammer des Ohrstöpsels kann ein Empfänger/Sender integriert sein, dessen abgestrahlten Audiosignale über ein innerhalb des Ohrstöpsels angeordnetes Audiofilter und eine einen Audiokanal bildende Bohrung zum Ohr gelangt. Das Audiofilter ist dabei sowohl für die Außengeräusche als auch für die von dem Empfänger/Sender abgegebenen Signale wirksam.

In ähnlicher Weise offenbart EP 0 440 572 Al ein akustisches Filter mit einem Resonanzhohlraum, an den sich ein länglicher Kanal anschließt. Ein derartiges akustisches Filter kann mit einem Hörer ausgestattet sein, der benachbart zum Resonanzhohlraum angeordnet ist. Der Hörer kann über ein Kabel mit einem Sender/Empfänger verbunden sein, um eine Kommunikation zwischen mehreren, derartige akustische Filter tragenden Personen zu ermöglichen. Demgemäß ist in dem Gehäuse des akustischen Filters auch ein Mikrofon zur Übertragung der Stimme der das akustische Filter tragenden Person angeordnet.

Das Mikrofon ist ebenfalls mit dem außerhalb des akustischen Filters angeordneten Sender/Empfänger verbunden, um die Kommunikation auch in Gegenrichtung zu ermöglichen.

Der Erfindung liegt die Aufgabe zugrunde, eine Vorrichtung zur Vermeidung von Schlafstörungen mit einer verbesserten Wirksamkeit anzugeben.

Diese Aufgabe wird durch eine Vorrichtung mit den Merkmalen des Anspruchs 1 gelöst. Weiterbildungen und vorteilhafte Ausgestaltungen sind in den Unteransprüchen angegeben.

Die erfindungsgemäße Vorrichtung ermöglicht über die beiden schalldämmenden Ohrstöpsel, jeglichen Lärm von dem Hörorgan des Benutzers fernzuhalten. Die erfindungsgemäße Vorrichtung sieht vor, dass bei einer schnarchenden Person, die die erfindungsgemäße Vorrichtung benutzt, das Schnarchen erkannt wird und zu einer Übertragung eines Funksignals von der Funkstation an den im Ohrstöpsel vorhandenen Funkempfänger führt, sodass dieser das empfangene Signal umwandelt und Tonsignale aussendet, die von der schnarchenden Person gehört werden. Der Schnarcher nimmt diese Tonsignale bewusst oder unbewusst wahr und beendet das Schnarchen, indem er beispielsweise die Schlafposition ändert. Die Tonsignale werden zweckmäßigerweise so laut eingestellt, dass der Schnarcher geweckt oder wenigstens angehalten wird, seine Schlafposition zu ändern.

Für den an Schlafstörungen leidenden Benutzer bedeutet das, dass zunächst eine der wesentlichen Ursachen für Schlafstörungen, nämlich schlafstörender Lärm, beseitigt und ein tiefer und erholsamer Schlaf gewährleistet wird. Für andere als an Schlafstörungen leidende Benutzer können die erfindungsgemäßen schalldämmenden Ohrstöpsel auch zur Vermeidung von Lärmbelästigungen während der beruflichen Tätigkeit oder in der Freizeit dienen.

Eine vorteilhafte Ausgestaltung der Erfindung verfolgt den Zweck, dass die Vorrichtung gleichzeitig von mehreren Benutzern verwendbar ist, wobei jeder Benutzer über zwei schalldämmenden Ohrstöpsel verfügt und wenigstens in einem der Ohrstöpsel ein Funkempfänger mit einem Mittel zur Umwandlung von von einer Funkstation empfangender Funksignale in Tonsignale integriert ist. Gerade bei Personengruppen, die in gleichen Räumen oder Wohnungen übernachten, ist eine solche Weiterbildung sinnvoll.

Überdies ist es möglich, dass über die Geräuscherkennungseinrichtung vorher festlegbar ist, an welchen Benutzer der Vorrichtung ein bestimmtes Geräusch übertragen werden soll.

Bei schnarchenden Personen, die sich in ihren Schnarchgeräuschen gegenseitig beeinflussen, eignet sich die Vorrichtung, gezielt dem verursachenden Schnarcher nach Erkennung ein Tonsignal oder sein eigenes Original-Schnarchgeräusch zu übermitteln, sodass dieser das Schnarchen einstellt.

Als Funkempfänger ist vorzugsweise eine Miniaturfunkempfänger zu ge-brauchen. Dadurch sind besonders kleine Ausführungsfomren der Ohrstöpsel möglich, sodass die Ohrstöpsel nicht als Fremdkörper empfunden werden.

In einer vorteilhaften Ausführungsform der erfindungsgemäßen Vorrichtung kann zusätzlich wenigstens ein alarmgebendes Mittel an die Funkstation angeschlossen sein. Ferner kann die Geräuscherkennungseinrichtung zur Erkennung weiterer spezifischer Geräusche ausgebildet sein.

Die erfindungsgemäße Vorrichtung lässt den Benutzer ereignisabhängig sein Schnarchen, einen Alarm oder ein anderes Geräusch trotz der Schalldämmung im Übrigen wahrnehmen. Ereignisabhängig heißt, dass nicht jeder denkbare Alarm und/oder nicht jedes Geräusch in ein Funksignal umgewandelt und von einer Funkstation an den Funkempfänger gesendet wird, denn dies würde die gewünschte schalldämmende Wirkung der Ohrstöpsel beeinträchtigen.

Ein Ereignis ist somit nur ein bestimmter Alarm oder ein anderes gewollt wahrnehmbares Geräusch, das dann von der Funkstation in Funksignale umgewandelt und an den im Ohrstöpsel integrierten Funkempfänger übertragen wird.

Durch die erfindungsgemäße Vorrichtung werden bei einem an Schlafstörungen leidenden Benutzer sowohl schlafstörender Lärm als auch andere psychisch bedingte Ursachen für Schlafstörungen, wie die Angst, wichtige Geräusche zu überhören oder zu Schnarchen, beseitigt.

Durch die erfindungsgemäße Vorrichtung wird ein großer Teil der schlafstörenden Ängste abgebaut, sodass sehr viele Menschen wieder ruhig und tief schlafen können.

Eine Weiterbildung sieht vor, dass der Ohrstöpsel aus im Außenohr individuell ausgeformten oder aufgeschäumten Silikon oder silikonhänlichem Material besteht, in das der Funkempfänger mit dem Mittel zur Umwandlung der von der Funkstation empfangenen Funksignale in Tonsignale eingegossen oder eingeschäumt oder austauschbar eingebaut ist.

Durch die exakte Anpassung der Ohrstöpsel an die betreffenden Teile des Ohres des Benutzers wird eine sehr gute Schalldämmung erreicht. Insbesondere die Verwendung von Silikon oder silikonähnlichem Material hat sich als sehr vorteilhaft für die Schalldämmung und zur Aufnahme des Funkempfängers und des Mittels zur Umwandlung der von der Funkstation empfangenen Funksignale in Tonsignale erwiesen. Dabei liefert eine Membran als Mittel zur Umwandlung der von der Funkstation empfangenen Funksignale in Tonsignale zuverlässige Ergebnisse.

Weiterhin ist vorgesehen, dass die Art der Tonsignale, insbesondere die Lautstärke, vorher festlegbar ist.

Dadurch wird erreicht, dass die Tonsignale den individuellen Bedürfnissen und Vorlieben der Benutzer angepasst werden können.

Unter den schlafenden und/oder an Schlafstörungen leidenden Benutzern gibt es beispielsweise Personen mit einem besonders leichten Schlaf. Hier ist die Möglichkeit gegeben, Tonsignale mit einer relativ geringen Lautstärke zu wählen. Außerdem haben manche Benutzer der erfindungsgemäßen Vorrichtung ein ausgeprägteres Gehör für ganz bestimmten Tonhöhen. Auch hier lassen sich entsprechende Anpassungen vornehmen.

Überdies ist vorstellbar, dass die Vorrichtung in einer sehr geräuschintensiven Umgebung benutzt wird. Da die Ohrstöpsel
die dort auftretenden Geräusche dämmen, aber nicht unbedingt beseitigen, ist es möglich, Tonsignale zu wählen, die entweder sehr laut sind oder sich in geeigneter Weise von denen der umgebenden Geräusche unterscheiden.

Eine vorteilhafte Ausgestaltung der Erfindung sieht vor, dass die Tonsignale den Alarm und/oder das Schnarchen identisch wiedergeben.

Dies hat den Vorteil, dass der Benutzer der erfindungsgemäßen Vorrichtung die wahrgenommenen Tonsignale sofort ihrem Ursprung zuordnen kann. Das ist insbesondere dann wichtig, wenn es sich bei den wahrgenommenen Tonsignalen um einen Alarm handelt, auf den möglichst schnell reagiert werden muss.
Das Mikrofon und ein etwaiges zusätzliches alarmgebendes Mittel kann unmittelbar oder über Funk an die Funkstation angeschlossen sein.

Dadurch wird erreicht, dass sowohl sehr nah an der Funkstation als auch sehr weit entfernt von der Funkstation befindliche alarmgebende Mittel mit der Funkstation gekoppelt werden können. Somit ist es möglich, praktisch jeden gewollt wahrnehmbaren Alarm dem Benutzer der Vorrichtung zuzuleiten.

Eine Integration des Mikrofons und etwaiger alarmgebender Mittel verfolgt den Zweck, die gesamte Vorrichtung auf relativ kleine Maße zu reduzieren. Vorteilhaft lässt sich dadurch die erfindungsgemäße Vorrichtung transportieren und an beliebigen Orten benutzen.

Eine Weiterbildung sieht vor, dass das alarmgebende Mittel eine digitale oder analoge Uhr mit Weckfunktion ist.

Durch diese Weiterbildung wird erreicht, dass die Benutzer der erfindungsgemäßen Vorrichtung die Vorteile der Schalldämmung nutzen können, ohne Angst haben zu müssen, wichtige Termine zu verpassen.

Bei den schlafenden und/oder an Schlafstörungen leidenden Personen werden die durch Lärm und andere Geräusche bedingten Schlafstörungen und die psychisch bedingten Schlafstörungen, zu denen die Angst gehört, wichtige Termine zu verpassen, beseitigt.

Aber auch bei anderen als an Schlafstörungen leidenden Personen liegt mit der wichtigste Grund, warum sich viele Personen vor der Benutzung von schalldämmenden Ohrstöpsel scheuen, darin, dass sie Angst haben, den Alarm einer Uhr zu überhören und dadurch wichtige Termine zu verpassen.

Das etwaige zusätzliche alarmgebende Mittel kann eine Türklingelstation, eine Babyüberwachungseinrichtung, ein Rauchmelder oder eine ähnliche, einen Audioalarm auslösende Vorrichtung sein. Ebenso kann das alarmgebende Mittel das Empfangsteil eines Bewegungsmelders sein.

Die zusätzlichen alarmgebenden Mittel können beispielsweise von einem Einbrecher erzeugte Geräusche detektieren. Bei Benutzung sonst üblicher Ohrstöpsel können diese Geräusche sehr leicht überhört werden.

Die erfindungsgemäße Geräuscherkennungseinichtung ist dafür vorgesehen, das Schnarchen einer bestimmten Person bzw. mehrerer bestimmter Personen zu speichern. Bei Übereinstimmung der aufgenommenen mit den gespeicherten Schnarchgeräuschen werden entsprechende Funksignale an den Funkempfänger übertragen.

Nachfolgend wird die Erfindung anhand eines Ausführungsbeispiels erläutert, das in der Zeichnung dargestellt ist. In dieser zeigt:
- Fig. 1: eine schematische Darstellung der erfindungsgemäßen Vorrichtung zur Schalldämmung am menschlichen Ohr
- Fig. 2: ein Blockschaltbild einer Funkstation und
- Fig. 3: ein Blockschaltbild eines Miniaturfunkempfängers.

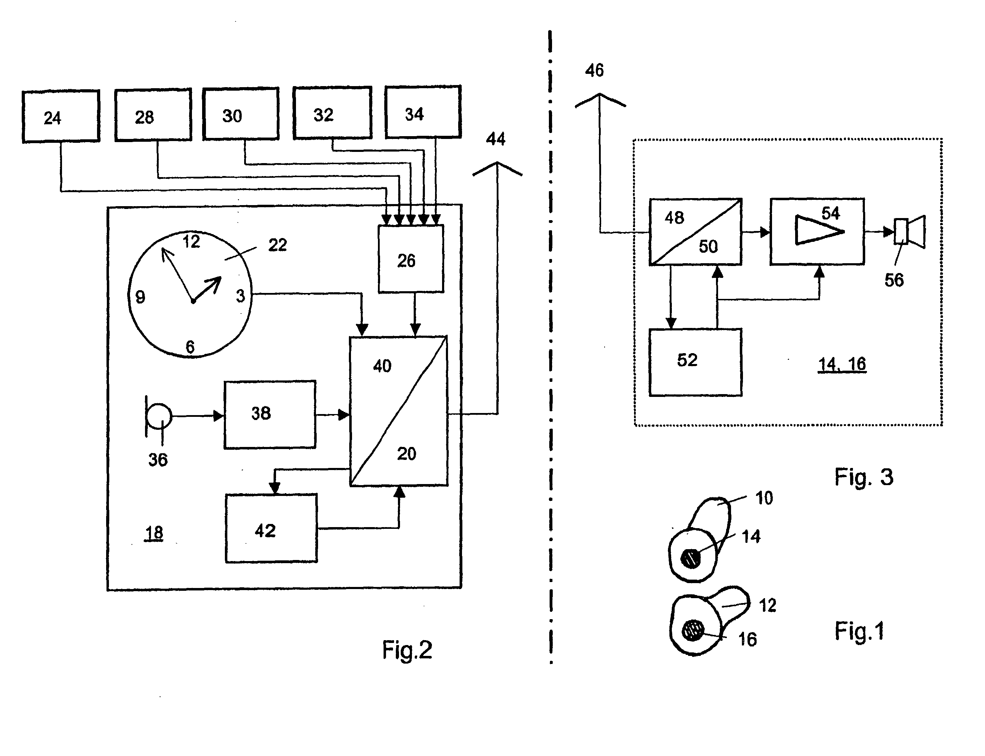

Die in Fig. 1 dargestellte Vorrichtung zur Schalldämmung am menschlichen Ohr umfasst zwei Ohrstöpsel 10, 12, in die je ein Miniaturfunkempfänger 14, 16 integriert ist. Jeder Ohrstöpsel 10, 12 ist aus Silikon und individuell der Form der betreffenden Teile eines Ohres des jeweiligen Benutzer angepasst, da das Silikon in der Ohrmuschel aufgeschäumt wird. Dadurch und durch die schalldämmende Wirkung von Silikon wird sichergestellt, dass keine störenden Geräusche von den Hörorganen des Benutzers der erfindungsgemäßen Vorrichtung aufgenommen werden können.

Die in den Ohrstöpseln 10, 12 eingeschäumten Miniaturfunkempfänger 14, 16 lösen nach dem Empfang eines Funksignals mittels eines Wandlers bestimmte Tonsignale aus, die von den Hörorganen des Benutzers wahrgenommen werden.

Fig. 2 zeigt ein Blockschaltbild einer Funkstation 18 mit einem Modulator 40, einem elektronischen Schalter 42, einem Sender 20, vorzugsweise für den Gigahertz-Bereich, und einer Antenne 44. In die Funkstation 18 integriert sind eine Uhr 22 mit Weckfunktion und ein Mikrofon 36. Zwischen Mikrofon 36 und Modulator 40 ist ein Mittel zur Geräuscherkennung 38 von aufgenommenen Geräuschen angeordnet. Außerdem ist in der Funkstation 18 ein Empfangsteil 26 für externe Alarmmodule angeordnet. Als Beispiel sind ein Bewegungsmelder 24, ein Telefon 28, eine Türklingelstation 30, eine Babyüberwachungseinrichtung 32 und ein Rauchmelder 34 dargestellt und angeschlossen. Ein Anschluss kann auch drahtlos über eine Funk-, Infrarot- oder Ultraschallstrecke erfolgen.

Fig. 3 zeigt ein Blockschaltbild eines Miniaturfunkempfängers 14, 16, wie er in den Ohrstöpseln 10, 12 angeordnet ist. Jeder Miniaturfunkempfänger 14, 16 umfasst eine Antenne 36, einen Empfänger 48 für den Gigahertz-Bereich mit einem Demodulator 50, einen Verstärker 54, einen elektronischen Schalter 52 und einen Schallwandler 56. Die empfangenen demodulierten Tonsignale werden so nach Verstärkung vom Schallwandler 56 in Tonsignale umgewandelt. Der elektronische Schalter 52 dient dazu, den Verstärker 54 stimm zu schalten, wenn kein Signal von der Funkstation 18 empfangen wird. Dadurch wird störendes Rauschen unterdrückt.

Bei Benutzung der erfindungsgemäßen Vorrichtung zur Schalldämmung am menschlichen Ohr befinden sich die Miniaturfunkempfänger 14, 16 im Empfangsbereich der Funkstation 18. Wenn an der Uhr 22 eine bestimmte Weckzeit eingestellt ist, wird ein Wecksignal von der Funkstation 18 übertragen, das von dem Miniaturfunkempfänger 14, 16 aufgenommen und vom Schallwandler 56 in ein Tonsignal umgewandelt wird, das vom Ohr wahrgenommen wird.

Für einen schlafenden und/oder an Schlafstörungen leidenden Benutzer der erfindungsgemäßen Vorrichtung ist es auf diese Weise möglich, tief und ungestört, das heißt ohne jegliche Lärmbeeinflussung, zu schlafen und dennoch durch den Weckruf der Uhr 22 zuverlässig geweckt zu werden. Auch die durch Ängste hervorgerufenen Schlafstörungen, beispielsweise die Angst, einen wichtigen Termin zu verschlafen, werden hierdurch beseitigt.

Die Funkstation 18 ist des weiteren mit dem Empfangsteil 26 für unterschiedliche Alarmmodule gekoppelt. Dies kann ein Bewegungsmelders 24 sein. Beim unbefugten Öffnen einer Tür, eines Fensters oder Bewegungen von unbefugten Personen wird von dem Empfangsteil 26 ein Signal des Bewegungsmelder 24 aufgenommen und an die Funkstation 18 übertragen. Von der Funkstation 18 wird daraufhin über den Sender 20 ein spezifisches Funksignal ausgesendet, das von dem Miniaturfunkempfänger 14, 16 im Ohrstöpsel 10, 12 empfangen und in ein Tonsignal für den schlafenden Benutzer umgewandelt wird.

Auf ähnliche Weise können auch Geräusche, die durch ein Telefon 28, eine Türklingelstation 30, eine Babyüberwachungseinrichtung 32 oder einen Rauchmelder 34 erzeugt werden, in entsprechende Funksignale umgewandelt werden. Auch Geräusche, die bei einem Wohnungseinbruch verursacht und von dem in der Funkstation 18 integrierten Mikrofon 36 aufgenommen werden, können in entsprechende Funksignale umgewandelt werden. Diese Funksignale werden dann von dem Miniaturfunkempfänger 14, 16 aufgenommen und dort in ein vom Benutzer wahrnehmbares Tonsignal oder Wecksignal umgewandelt werden. Ein elektronischer Schalter 42 dient dazu, den Sender 20 nur dann einzuschalten, wenn Geräusche oder Alarm zu den Miniaturfunkempfängern 14, 16 übertragen werden sollen.

Die erfindungsgemäße Vorrichtung eignet sich ferner besonders gut zur Beseitigung von durch Schnarchen bedingten Schlafstörungen.

Über ein in der Funkstation 18 integriertes Mittel zur Aufnahme von Geräuschen, das Mikrofon 36, werden die Schnarchgeräusche des die Vorrichtung benutzenden Schnarchers aufgenommen. Wenn das Schnarchen ein vorher festlegbares Ereignis darstellt, das gesondert ausgewertet und identifiziert werden kann, dann wird von der Funkstation 18 ein Funksignal an den Miniaturfunkempfänger 14, 16 des Schnarchers gesendet. Der Schnarcher nimmt die entsprechenden Tonsignale wahr. Die Lautstärke der Tonsignale ist für diesen Fall so gewählt, daß der Schnarcher entweder geweckt oder dazu angehalten wird, seine Schlafposition zu ändern und dadurch das Schnarchen einzustellen.

Als Tonsignale können neben künstlichen Geräuschen auch die original Schnarchgeräusche identisch wiedergegeben werden, so daß die schnarchende Person in einer Art Geräuschrückkopplung ihr eigenes Schnarchen hört und den Schnarchvorgang unterbricht.

Es hat sich gezeigt, daß der Schnarcher bei häufiger Anwendung der erfindungsgemäßen Vorrichtung entsprechend sensibilisiert wird und automatisch schon beim Einschlafen eine nicht schnarchgefährdete Schlafposition einnimmt.

Zur Überprüfung, ob das Schnarchen oder ein anderes Geräusch ein Ereignis darstellt, ist zwischen dem Mikrofon 36 und dem Modulator 18 eine Geräuscherkennung 38 angeordnet. Die Geräuscherkennung 38 kann dabei auch in der Funkstation 18 integriert sein. Durch die Erkennung werden nur bestimmte vorher festlegbare Geräusche von der Funkstation 18 zum Funkempfänger 14, 16 gesendet.

Dazu dient eine in der Funkstation integrierte hard- und/oder softwaregestützte Sprach- oder Schnarcherkennung. Die aufgenommenen Schnarchgeräusche können dadurch beispielsweise dem Verursacher zugeordnet werden, um bei einem erneuten Schnarchen die Weiterleitung dieser Geräusche an den Miniaturfunkempfänger 14, 16 der schnarchenden Person zu veranlassen. Dadurch wird das Schnarchen schon frühzeitig unterbunden.

Bei mehreren Benutzern der Vorrichtung ist es über die hardund/oder softwaregestützte Sprach- oder Schnarcherkennung natürlich auch möglich, Funksignale nur an den Schnarchgeräusche verursachenden Benutzer zu übertragen.

Überdies werden mittels der erfindungsgemäßen Vorrichtung die Schlafstörungen der Partner von Schnarchenden beseitigt. Jene lehnen die Benutzung der bereits bekannten Mittel zur Schalldämmung aus Angst ab, Phasen der Atemlosigkeit des schnarchenden Partners zu überhören und dann bei einem längeren Atemstillstand nicht reagieren zu können. Über die hardund/oder softwaregestützte Sprach- oder Schnarcherkennung ist es möglich, die Zeiträume zwischen einzelnen Schnarchgeräuschen zu messen und damit die Atmung des Schnarchenden zu überwachen. Bei einem lebensbedrohlich langen Atemstillstand, wäre dann die Möglichkeit gegeben, den Partner der schnarchendenden Person über ein Tonsignal zu alarmieren.

Es versteht sich, daß die erfindungsgemäßen Ohrstöpsel 10, 12 unabhängig von der Funkstation 18 auch von nicht schlafenden Personen benutzt werden können, um bei großer Lärmbelästigung während der Freizeit oder bei beruflicher Tätigkeit einen Lärmschutz zu erzielen. In Verbindung mit der oben beschriebenen Funkstation 18 können von den gegenüber Lärmeinwirkung abgeschirmten Personen dennoch bestimmte Signale, zum Beispiel Warnsignale bei Bauarbeiten, gehört werden.

Die Erfindung ist nicht auf das vorstehend beschriebene Ausführungsbeispiel beschränkt. Vielmehr sind im Rahmen des Grundgedankens der Erfindung, der in der Bereitstellung von mit einem Miniaturfunkempfänger 14, 16 versehenen schalldämmenden Ohrstöpsel 10, 12 besteht, die ereignisabhängig bestimmte Signale von einer Funkstation 18 empfangen und bestimmte Tonsignale im Ohr erzeugen können, wobei die Funkstation 18 an alarmgebende und/oder Geräusche aufnehmende Mittel 24 - 34 angeschlossen ist, zahlreiche Modifikationen denkbar.

Zielgruppen sind unter anderen alle nachts arbeitenden Notdienste, Gefängnispersonal, Feuerwehr, Krankenpfleger und - schwestern, Ärzte, sonstige Pflegedienste, Wachdienste, Polizei, Katastrophenschutzverbände, Bundeswehreinheiten, Bereitschaftsdienste jeder Art oder alle tagsüber arbeitenden Menschen, die einem erhöhten Geräuschpegel ausgesetzt sind.

### Bezugszeichenliste

- 10.: Ohrstöpsel
- 12.: Ohrstöpsel
- 14.: Miniaturfunkempfänger
- 16.: Miniaturfunkempfänger
- 18.: Funkstation
- 20.: Sender
- 22.: Uhr
- 24.: Bewegungsmelders
- 26.: Empfangsteil
- 28.: Telefon
- 30.: Türklingelstation
- 32.: Babyüberwachungseinrichtung
- 34.: Rauchmelder
- 36.: Mikrofon
- 38.: Geräuscherkennung
- 40.: Modulator
- 42.: elektronischer Schalter
- 44.: Antenne
- 46.: Antenne
- 48.: Empfänger
- 50.: Demodulator
- 52.: elektronischer Schalter
- 54.: Verstärker
- 56.: Schallwandler

## Patentansprüche

1. Vorrichtung zur Vermeidung von durch Lärm und andere Geräusche bedingten Schlafstörungen mit einer Funkstation (18) und zwei schalldämmenden Ohrstöpseln (10, 12), von denen wenigstens einer einen integrierten Funkempfänger (14, 16) mit einem Mittel zur Umwandlung von empfangenen, von der Funkstation (18) ausgehenden Funksignalen in Tonsignale aufweist, **dadurch gekennzeichnet, dass** die Funkstation (18) ein Mikrofon (36) und eine zur Erkennung von Schnarchgeräuschen eingerichtete Geräuscherkennungseinrichtung (38) aufweist und durch den Empfang eines Schnarchgeräusches zur Aussendung eines vom Funkempfänger (14, 16) empfangbaren und in ein Tonsignal umwandelbaren Funksignals schaltbar ist.

2. Vorrichtung nach Anspruch 1, **dadurch gekennzeichnet, dass** das Funksignal nach Umwandlung im Funkempfänger (14, 16) als Tonsignal Schnarchgeräusche darstellt.

3. Vorrichtung nach Anspruch 1 oder 2, **dadurch gekennzeichnet, dass** zusätzlich wenigstens ein alarmgebendes Mittel (22 bis 34) an die Funkstation (18) angeschlossen ist.

4. Vorrichtung nach einem der Ansprüche 1 bis 3, **dadurch gekennzeichnet, dass** die Geräuscherkennungseinrichtung (38) zur Erkennung weiterer spezifischer Geräusche ausgebildet ist.

5. Vorrichtung nach einem der Ansprüche 1 bis 4, **dadurch gekennzeichnet, dass** ein Ohrstöpsel (10, 12) aus im Außenohr individuell ausgeformten oder aufgeschäumten Silikon oder silikonähnlichem Material besteht, in das der Funkempfänger (14, 16) mit dem Mittel zur Umwandlung von der Funkstation (18) empfangener Funksignale in Tonsignale eingegossen, eingeschäumt oder austauschbar eingebaut ist.

6. Vorrichtung nach einem der Ansprüche 1 bis 5, **dadurch gekennzeichnet, dass** die Art der Tonsignale, insbesondere die Lautstärke, vorher festlegbar ist.

7. Vorrichtung nach einem der Ansprüche 1 bis 6, **dadurch gekennzeichnet, dass** die Tonsignale den Alarm und/oder das Geräusch identisch wiedergeben.

8. Vorrichtung nach einem der Ansprüche 1 bis 7, **dadurch gekennzeichnet, dass** das Mikrofon (36) und ein etwaiges zusätzliches alarmgebendes Mittel (22 bis 34) unmittelbar oder über Funk an die Funkstation (18) angeschlossen ist.

9. Vorrichtung nach einem der Ansprüche 1 bis 8, **dadurch gekennzeichnet, dass** das Mikrofon (36) und ein etwaiges zusätzliches alarmgebendes Mittel (22 bis 34) in die Funkstation (18) integriert sind.

10. Vorrichtung nach einem der Ansprüche 3 bis 8, **dadurch, gekennzeichnet, dass** das zusätzliche alarmgebende Mittel eine digitale oder analoge Uhr (22) mit Weckfunktion ist.

11. Vorrichtung nach einem der Ansprüche 3 bis 10, **dadurch gekennzeichnet, dass** das zusätzliche alarmgebende Mittel ein Telefon (28), eine Türklingelstation (30), eine Babyüberwachungseinrichtung (32), ein Rauschmelder (34) oder eine ähnliche, einen Audioalarm auslösende Vorrichtung ist .

12. Vorrichtung nach einem der Ansprüche 3 bis 11, **dadurch gekennzeichnet, dass** das zusätzliche alarmgebende Mittel das Empfangsteil (26) eines Bewegungsmelders (24) ist.

13. Vorrichtung nach einem der Ansprüche 1 bis 12, **dadurch gekennzeichnet, dass** die Vorrichtung gleichzeitig von mehreren Benutzern verwendbar ist, wobei jeder Benutzer über zwei schalldämmende Ohrstöpsel (10, 12) verfügt und wenigstens in einem der Ohrstöpsel (10, 12) ein Funkempfänger (14, 16) mit einem Mittel zur Umwandlung von einer Funkstation (18) empfangener Funksignale in Tonsignale integriert ist.

14. Vorrichtung nach Anspruch 13, **dadurch gekennzeichnet, dass** über die Geräuscherkennungseinrichtung (38) vorher festlegbar ist, an welchen Benutzer der Vorrichtung ein bestimmtes aufgenommenes Schnarchgeräusch übertragen wird.

## Claims

1. Apparatus for avoiding sleep disturbances which are caused by noise and other sounds, having a radio station (18) and two sound-attenuating earplugs (10, 12), at least one of which has an integrated radio receiver (14, 16) with a means for conversion of received radio signals, which originate from the radio station (18), to audio signals, **characterized in that** the radio station (18) has a microphone (36) and a sound identification device (38) which is designed to identify snoring sounds, and which can be switched by the reception of a snoring sound to transmit a radio signal which can be received by the radio receiver (14, 16) and can be converted to an audio signal.

2. Apparatus according to Claim 1, **characterized in that** the radio signal represents a snoring sound, after conversion in the radio receiver (14, 16) as an audio signal.

3. Apparatus according to Claim 1 or 2, **characterized in that**, in addition, at least one alarm-emitting means (22 to 34) is connected to the radio station (18).

4. Apparatus according to one of Claims 1 to 3, **characterized in that** the sound identification device (38) is designed to identify further specific sounds.

5. Apparatus according to one of Claims 1 to 4, **characterized in that** an earplug (10, 12) is composed of silicone, which is formed or expanded by foaming individually in the outer ear, or of a material similar to silicone, in which the radio receiver (14, 16) with the means for conversion of radio signals which are received from the radio station (18) to audio signals is encapsulated, foamed-in or installed replaceably.

6. Apparatus according to one of Claims 1 to 5, **characterized in that** the nature of the audio signals, in particular the volume, can be defined in advance.

7. Apparatus according to one of Claims 1 to 6, **characterized in that** the audio signals reproduce the alarm and/or the sound identically.

8. Apparatus according to one of Claims 1 to 7, **characterized in that** the microphone (36) and any additional alarm-emitting means (22 to 34) are connected directly or by radio to the radio station (18).

9. Apparatus according to one of Claims 1 to 8, **characterized in that** the microphone (36) and any additional alarm-emitting means (22 to 34) are integrated in the radio station (18).

10. Apparatus according to one of Claims 3 to 8, **characterized in that** the additional alarm-emitting means is a digital or analogue clock (22) with an alarm function.

11. Apparatus according to one of Claims 3 to 10, **characterized in that** the additional alarm-emitting means is a telephone (28), a doorbell station (30), a baby monitoring device (32), a smoke alarm (34) or some similar apparatus which triggers an audio alarm.

12. Apparatus according to one of Claims 3 to 11, **characterized in that** the additional alarm-emitting means is the receiving section (26) of a motion sensor (24).

13. Apparatus according to one of Claims 1 to 12, **characterized in that** the apparatus can be used at the same time by two or more users, with each user having two sound-attenuating earplugs (10, 12), and a radio receiver (14, 16) with a means for conversion of radio signals which are received from a radio station (18) to audio signals is integrated in at least one of the earplugs (10, 12).

14. Apparatus according to Claim 13, **characterized in that** it is possible to use the sound identification device (38) to determine in advance which user of the apparatus a specific received snoring sound will be transmitted to.

## Revendications

1. Dispositif pour éviter les troubles du sommeil causés par le bruit, comprenant une station radio (18) et deux bouchons d'oreille insonorisants (10, 12) dont l'un au moins comporte un récepteur radio intégré (14, 16) avec un moyen pour convertir en signaux sonores des signaux radio reçus émanant de la station radio (18), **caractérisé en ce que** la station radio (18) comprend un microphone (36) et un dispositif de reconnaissance des bruits (38) adapté pour la reconnaissance des bruits de ronflement, et peut être commutée par la réception d'un bruit de ronflement pour émettre un signal radio que le récepteur radio (14, 16) peut recevoir et convertir en un signal sonore.

2. Dispositif selon la revendication 1, **caractérisé en ce qu'**une fois converti dans le récepteur radio (14, 16) le signal radio représente des bruits de ronflement en tant que signal sonore.

3. Dispositif selon la revendication 1 ou 2, **caractérisé en ce qu'**en outre au moins un moyen d'alarme (22 à 34) est raccordé à la station radio (18).

4. Dispositif selon l'une des revendications 1 à 3, **caractérisé en ce que** le dispositif de reconnaissance des bruits (38) est réalisé pour reconnaître d'autres bruits spécifiques.

5. Dispositif selon l'une des revendications 1 à 4, **caractérisé en ce qu'**un bouchon d'oreilles (10, 12) est constitué d'un matériau silicone ou d'un matériau analogue à du silicone, formé individuellement ou expansé en mousse individuellement dans l'oreille externe, matériau dans lequel le récepteur radio (14, 16) avec le moyen pour convertir en signaux sonores les signaux radio reçus de la station radio (18) est noyé, intégré dans la mousse, ou installé de façon interchangeable.

6. Dispositif selon l'une des revendications 1 à 5, **caractérisé en ce que** le type de signaux sonores, en particulier le volume sonore est prédéterminable.

7. Dispositif selon l'une des revendications 1 à 6, **caractérisé en ce que** les signaux sonores restituent identiquement l'alarme et/ou le bruit.

8. Dispositif selon l'une des revendications 1 à 7, **caractérisé en ce que** le microphone (36) et un éventuel moyen d'alarme (22 à 34) additionnel sont raccordés directement ou par radio à la station radio (18).

9. Dispositif selon l'une des revendications 1 à 8, **caractérisé en ce que** le microphone (36) et un éventuel moyen d'alarme (22 à 34) additionnel sont intégrés dans la station radio (18).

10. Dispositif selon l'une des revendications 3 à 8, **caractérisé en ce que** le moyen d'alarme additionnel est une horloge numérique ou analogique (22) avec fonction réveil.

11. Dispositif selon l'une des revendications 3 à 10, **caractérisé en ce que** le moyen d'alarme additionnel est un téléphone (28), un poste de sonnette de porte (30), un dispositif de surveillance de bébé (32), un détecteur de bruit (34) ou un dispositif analogue déclenchant une alarme audio.

12. Dispositif selon l'une des revendications 3 à 11, **caractérisé** en ce le moyen d'alarme additionnel est la partie réceptrice (26) d'un détecteur de mouvement (24).

13. Dispositif selon l'une des revendications 1 à 12, **caractérisé en ce que** le dispositif est utilisable simultanément par plusieurs utilisateurs, chaque utilisateur disposant de deux bouchons d'oreille insonorisants (10, 12) et dans au moins un des bouchons d'oreilles (10, 12) est intégré un récepteur radio (14, 16) avec un moyen pour convertir en signaux sonores des signaux radio reçus d'une station radio (18).

14. Dispositif selon la revendication 13, **caractérisé en ce qu'**on peut déterminer à l'avance par l'intermédiaire du dispositif de reconnaissance de bruit (38) à quel utilisateur du dispositif est transmis un bruit de ronflement capté déterminé.
